# FASCICULE DE BREVET EUROPEEN

(11) **EP 0 923 369 B1**
(45) Date de publication et mention de la délivrance du brevet: **12.05.2004**
(21) Numéro de dépôt: 97930612.3
(22) Date de dépôt: 27.06.1997
(51) Int. Cl.: A61K 31/08, A01K 67/02, A01N 1/00, A61D 19/02, A61K 35/52, A61P 15/00

(54) **1-O-ALKYLGLYCEROLS A USAGE PHARMACEUTIQUE OU VETERINAIRE POUR CONSERVATION ET/OU AMELIORATION DU SPERME**
1-0-ALKYLGLYCEROLE ZUM PHARMAZEUTISCHEN ODER TIERÄRZTLICHEN GEBRAUCH ZUR KONSERVIERUNG UND/ODER VERBESSERUNG DES SAMENS
1-O-ALKYLGLYCEROLS FOR PHARMACEUTICAL OR VETERINARY USE FOR PRESERVING AND/OR IMPROVING SPERM

(30) Priorité: 28.06.1996 FR 9608314
(43) Date de publication de la demande: 23.06.1999
(73) Titulaire: UNIVERSITE DE RENNES I, F-35000 Rennes (FR)
(72) Inventeur: LEGRAND, Alain, F-22270 Jugon-les-Lacs (FR)
(74) Mandataire: Vidon, Patrice
(86) Numéro de dépôt international: PCT/FR1997/001160
(87) Numéro de publication internationale: WO 1998/000120

(56) Documents cités:
- EP-A- 0 321 428
- EP-A- 0 333 678
- EP-A- 0 335 844
- CHEMICAL ABSTRACTS, vol. 110, no. 25, 19 juin 1989 Columbus, Ohio, US; abstract no. 229248z, PARASHCHUK ET AL.: "the comparative estimation of cytotoxicity and cryoprotective efficiency of glycerol and its alkyl ethers upon freezing of human sperm" XP002030147 & PARASHCHUK ET AL.: KRIOBIOLOGIYA, vol. 1, 1989, pages 40-43,
- NISSEN ET AL.: "Stellenwert der Neutralfette als potentielle Energiequelle der menschlichen Spermatozoen" FETTE, SEIFEN, ANSTRICHMITTEL, vol. 2, 1983, pages 595-599, XP002030146

## Description

L'invention concerne le domaine pharmaceutique ainsi que le domaine vétérinaire.

Plus précisément, l'invention concerne le domaine de la reproduction, et trouve notamment son application dans le domaine du traitement de la stérilité chez l'être humain et dans le domaine de l'amélioration de la fertilité chez l'animal. L'invention trouve également son application dans le domaine de la fécondation in vitro et de la procréation assistée.

L'objectif de la présente invention est de proposer des utilisations nouvelles de composés connus en tant que tels, jouant un rôle dans le domaine de la reproduction. Plus précisément, un des objectifs de la présente invention est de proposer d'utiliser de tels composés pour lutter contre la stérilité chez l'homme.

Un autre objectif de l'invention est de décrire l'utilisation nouvelle de tels composés pour augmenter la fertilité des aimaux d'élevage, et ainsi augmenter la productivité de ces élevages.

Encore un autre objectif de la présente invention est de divulguer l'utilisation nouvelle de tels composés pour favoriser la fécondation in vitro ou encore pour permettre une meilleure conservation du sperme lors de la mise en oeuvre de procédés de procréation assistée.

Ces différents objectifs, ainsi que d'autres qui apparaîtront par la suite sont atteints grâce à l'invention qui concerne l'utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un médicament à usage humain destiné à lutter contre la stérilité masculine.

Selon la présente invention, il a en effet été découvert que de tels composés permettaient d'augmenter de façon sensible la motricité des spermatozoïdes et qu'ainsi ils pouvaient être utilisés pour lutter contre certains cas de stérilité masculine.

Selon une variante préférentielle, un tel médicament est conçu pour pouvoir être administré par voie orale de façon à présenter l'avantage de pouvoir être facilement absorbés et ainsi de faciliter le traitement dans lequel il s'inscrit.

L'invention concerne également l'utilisation d'un composé 1-O-alkylglycérol choisi dans le groupe constitué par le 16 :0 alkyl-1-glycérol, le 18 :1 alkyl-1-glycérol et le 16:1 alkyl-1-glycérol, pour la fabrication d'un adjuvant alimentaire à usage vétérinaire destiné à augmenter les rendements de fertilisation dans les élevages.

Cette utilisation nouvelle est également basée sur la potentialité de tels composés à favoriser la motricité des spermatozoïdes.

L'invention concerne aussi l'utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un adjuvant de conservation du sperme. Un tel composé permet ainsi d'améliorer le pouvoir fécondant du sperme conservé ce qui permet d'augmenter les chances de succès lors de la mise en oeuvre de procédés procréation assistée.

Enfm, l'invention concerne également l'utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un facteur favorisant la mobilité des spermatozoïdes lors de la fécondation in vitro. Un tel facteur est susceptible d'augmenter les chances de succès de l'opération de fécondation in vitro.

Préférentiellement le composé 1-O-alkylglycérol utilisé pour toutes ces applications nouvelle est d'origine naturelle. Les alkylglycérols existent en effet en petites quantités dans plusieurs produits naturels. On les trouve dans les organes hématopoïétiques particulièrement dans la moëlle osseuse, et aussi en concentration relativement importante dans le lait maternel. Toutefois, c'est dans les huiles de foie de certains poissons et en particulier de certines espèces de requins que ces etherlipides sont les plus abondants. Ces huiles contiennent en effet plus de 50% d'alkylglycérols.

C'est la raison pour laquelle le composé utilisé provient préférentiellement, d'huile de foie de poisson et avantageusement, d'huile de foie de requin. L'invention propose donc une voie de valorisation intéressante de ce type de produits issus de l'industrie de la pêche et qui présentent un coût traditionnellement faible.

Le composé 1-O- alkylglycérol est choisi dans le groupe constitué par par le 18:1 alkyl- 1 - glycérol, le 16:1 alkyl -1- glycérol et 16:0 alkyl- 1 - glycérol. Ces trois composés sont en effet ceux qui sont présents en plus grandes quantités dans les huiles de foie de requin, en particulier le 18:1 alkyl- 1 - glycérol.

L'activité des 1-O-alkylglycérol dans le cadre des utilisations mentionnées ci-dessus résulte du fait que de tels composés sont des précurseurs du "Platelet Activating Factor" (PAF).

Le "Platelet Activating Factor" (PAF) est un terme général qui regroupe les dérivés de la glycérophosphocholine (GPC). L'alkyl-PAF est le composé le plus actif, sa structure générale est 1-*O* -alkyl-2-acétyl-*sn* -glycéro-3-phosphocholine. Le nombre d'atomes de carbones et le degré d'insaturation de la chaîne alkyl peuvent varier sans modifier de façon importante l'activité biologique. Les alkényl-PAF ou plasmalogènes PAF (1-O-alk-1'-ényl-2-acétyl -*sn*-glycéro-3-phosphocholine) et les acyl-PAF (1-acyl-2-acétyl-*sn*-glycéro- 3-phosphocholine) sont beaucoup moins puissants que les alkyl-PAF. Le PAF possède un large éventail d'activités biologiques à des concentrations très basses (de 10⁻¹⁰ à 10⁻⁹ M). Initialement identifié comme un médiateur libéré par les leucocytes et capable d'activer les plaquettes sanguines, il a été établi que le PAF jouait un rôle dans le domaine de la reproduction. La production du PAF a ainsi été mise en évidence au cours de l'ovulation (Abisogun A.O. et al., (1989) Science 243:381-383). Il a également été démontrés que le PAF augmentait la motricité des spermatozoïdes de diverses espèces animales et de l'homme et que le traitement des spermatozoïdes par le PAF augmentait leur pouvoir de fertilisation des ovocytes de lapin ou de souris (Roudebush et al., (1993) J. Assis. Reprod. Gen. 10:91-94 ; Brijinder et al., (1989) Am. J. Obstet. Gynecol. 161:1714-1717).

On notera toutefois, que ces différents travaux ne se sont pas attachés à la possibilité d'utiliser les précurseurs du PAF pour des utilisations telles que proposées par l'invention.

On notera également qu'il avait déjà été proposé dans l'état de la technique d'utiliser le PAF lui-même pour tenter améliorer les propriétés du sperme conservé. Toutefois, une telle utilisation s'était heurté à un échec résultant du maniement délicat impliqué par la très grande activité biologique de ce produit.

Le document CHEMICAL ABSTRACTS vol. 110, no. 25, Abstract no. 229248z, 19-06-1989, PARASHCHUK ET AL. : "The comparative estimation of cytotoxicity and cryoprotective efficiency of glycerol and its alkyl ethers upon freezing of human sperm" décrit un effet cryoprotecteur du 1-méthylglycérol et du 1-éthylglycérol inférieur à celui du glycérol dans la conservation du sperme. Ce document ne divulgue pas de composé 16:0 alkyl-1-glycérol, 18:1 alkyl-1-glycérol ou 16:1 alkyl-1-glycérol.

Dans le but de confirmer l'activité des pour 1-O-alkylglycérols choisi dans le groupe constitué par le 16 :0 alkyl-1-glycérol, le 18 :1 alkyl-1-glycérol et le 16 :1 alkyl-1-glycérol, pour les applications spécifiques ci-dessus mentionnées, différents tests ont été effectués.

Une première série de test a été mise en oeuvre afin de vérifier que ces composés étaient bien intégrés dans les spermatozoïdes.

Dans ce but, différents échantillons de sperme humain, de sperme de verrat et de sperme de lapin ont été placés en incubation pendant 24 heures à 35 °C, en présence d'un extrait d'huile de foie de requin comprenant mélange d'1-O-alkylglycérols marqués au tritium (lesdits échantillons comprenant : 54 à 65 % de 18:01 alkyl-1-glycérol, 5 % à 15 % de 16:1 alkyl-1-glycérol et 5% à 10 % de 16:0 alkyl-1-glycérol ainsi que d'autres alkyiglycérols en quantités moindres). Ces tests ont été effectués en utilisant une concentration dudit mélange de 10⁻⁵ Mol/l d'1-O-alkylglycérols.

A l'issue de cette incubation, la fraction lipidique des spermatozoïdes a été extraite et la radioactivité de cette fraction a été mesurée. Les résultats montrent que :
- environ 12 % de la radioactivité initiale est retrouvée dans les lipides totaux des spermatozoïdes de verrat ;
- environ 10 % de la radioactivité initiale est retrouvée dans les lipides totaux des spermatozoïdes de lapin ;
- environ 5 % de la radioactivité initiale est retrouvée dans les lipides totaux des spermatozoïdes humains.

Ces résultats montrent bien l'intégration cellulaire des 1-O-alkylglycérols selon l'invention dans les spermatozoïdes.

Une étude plus précise a montré qu'une faible fraction de cette radioactivité était incorporée dans les phospholipides (phosphatidylcholine et phosphatidyléthanolamine), à savoir 0,1 % dans chacune de ces classes chez le verrat et le lapin, et moins de 1 % chez l'homme. Dans les lipides neutres, une très faible fraction est métabolisée sous forme de 1-alkyl-2-3-diacylglycérols (moins de 0,1 %) pour les trois espèces. Le reste de la radioactivé est trouvée sous forme native d'1-O-alkylglycérol.

Une deuxième série de tests a été effectuée et a permis de démontrer que la motilité des spermatozoïdes de verrat se trouvait sensiblement augmentée après incubation de 24 à 96 heures en présence du mélange d'alkyglycérol ci-dessus mentionné à raison de 10⁻⁵ Mol/l d'1-O-alkylglycérols.

## Revendications

1. Utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un médicament à usage humain destiné à lutter contre la stérilité masculine.

2. Utilisation selon la revendication 1 **caractérisé en ce que** ledit médicament est administrable par voie orale.

3. Utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un adjuvant alimentaire à usage vétérinaire destiné à augmenter les rendements de fertilisation dans les élevages.

4. Utilisation d'un composé 1-O-alkylglycérol choisi dans le groupe constitué par le 16 :0 alkyl-1-glycérol, le 18 :1 alkyl-1-glycérol et le 16 :1 alkyl-1-glycérol, pour la fabrication d'un adjuvant de conservation du sperme.

5. Utilisation d'un composé 1-O-alkylglycérol choisi dans le groupe constitué par le 16 :0 alkyl-1-glycérol, le 18 :1 alkyl-1-glycérol et le 16:1 alkyl-1-glycérol, pour la fabrication d'un facteur favorisant la mobilité des spermatozoïdes lors de la fécondation in vitro.

6. Utilisation selon l'une des revendications 1 à 5 **caractérisé en ce que** ledit composé 1-O-alkylglycérol est d'origine naturelle.

7. Utilisation selon la revendication 6 **caractérisé en ce que** ledit composé 1-O-alkylglycérol provient d'huile de foie de poisson.

8. Utilisation selon la revendication 7 **caractérisé en ce que** ledit composé 1-O-alkylglycérol provient d'huile de foie de requin.

## Patentansprüche

1. Verwendung einer 1-O-Alkylglycerin-Verbindung, ausgewählt aus der Gruppe bestehend aus 16:0 Alkyl-1-glycerin, 18:1 Alkyl-1-glycerin und 16:1 Alkyl-1-glycerin, zum Herstellen eines Medikaments zur Verwendung am Menschen, das bestimmt ist zur Behandlung der männlichen Sterilität.

2. Verwendung nach Anspruch 1, **dadurch gekennzeichnet, dass** das Medikament auf oralem Weg verabreichbar ist.

3. Verwendung einer 1-O-Alkylglycerin-Verbindung, ausgewählt aus der Gruppe bestehend aus 16:0 Alkyl-1-glycerin, 18:1 Alkyl-1-glycerin und 16:1 Alkyl-1-glycerin, zum Herstellen eines Nahrungsergänzungsmittels zur Verwendung am Tier, das bestimmt ist zum Verbessern der Fertilisationsausbeute in der Viehzucht.

4. Verwendung einer 1-O-Glykolglycerin-Verbindung, die ausgewählt ist aus der Gruppe bestehend aus 16:0 Alkyl-1-glycerin, 18:1 Alkyl-1-glycerin und 16:1 Alkyl-1-glycerin zum Herstellen eines Hilfsmittels zur Konservierung von Sperma.

5. Verwendung einer 1-O-Glykolglycerin-Verbindung, die ausgewählt ist aus der Gruppe bestehend aus 16:0 Alkyl-1-glycerin, 18:1 Alkyl-1-glycerin und 16:1 Alkyl-1-glycerin zum Herstellen eines die Mobilität von Spermien während der in vitro-Befruchtung begünstigenden Faktors.

6. Verwendung nach einer der Ansprüche 1 bis 5, **dadurch gekennzeichnet, dass** die 1-O-Alkylglycerin-Verbindung natürlichen Ursprungs ist.

7. Verwendung nach Anspruch 6, **dadurch gekennzeichnet, dass** die 1-O-Alkylglycerin-Verbindung aus Fischleberöl stammt.

8. Verwendung nach Anspruch 7, **dadurch gekennzeichnet, dass** die 1-O-Alkylglycerin-Verbindung aus Haifischleberöl stammt.

## Claims

1. Use of a 1-O-alkylglycerol compound chosen from the group consisting of 16:0 alkyl-1-glycerol, 18:1 alkyl-1-glycerol and 16:1 alkyl-1-glycerol, for the production of a medication for human use designed to combat masculine sterility.

2. Use according to claim 1, **characterized in that** the said medication can be administered orally.

3. Use of a 1-O-alkylglycerol compound chosen from the group consisting of 16:0 alkyl-1-glycerol, 18:1 alkyl-1-glycerol and 16:1 alkyl-1-glycerol, for the production of a food additive for veterinary use designed to increase fertilisation yields in stock breeding.

4. Use of a 1-O-alkylglycerol compound chosen from the group consisting of 16:0 alkyl-1-glycerol, 18:1 alkyl-1-glycerol and 16:1 alkyl-1-glycerol, for the production of an additive for preserving sperm.

5. Use of a 1-O-alkylglycerol compound chosen from the group consisting of 16:0 alkyl-1-glycerol, 18:1 alkyl-1-glycerol and 16:1 alkyl-1-glycerol, for the production of a factor promoting the mobility of spermatozoids during in vitro fertilisation.

6. Use according to one of claims 1 to 5, **characterized in that** the said 1-O-alkylglycerol compound is of natural origin.

7. Use according to claim 6, **characterized in that** the said 1-O-alkylglycerol compound is derived from fish liver oil.

8. Use according to claim 7, **characterized in that** the said 1-O-alkylglycerol compound is derived from shark liver oil.
